Europäisches Patentamt

European Patent Office

Office européen des brevets

(19)

(11) Veröffentlichungsnummer : **0 166 100**
**B1**

(12)

# EUROPÄISCHE PATENTSCHRIFT

(45) Veröffentlichungstag der Patentschrift :
**11.01.89**

(21) Anmeldenummer : **85104563.3**

(22) Anmeldetag : **15.04.85**

(51) Int. Cl.⁴ : **A 61 K   7/13**

(54) **Oxidationshaarfärbemittel auf Basis einer niedrigviskosen Trägermasse.**

(30) Priorität : **27.06.84 DE 3423589**

(43) Veröffentlichungstag der Anmeldung :
**02.01.86 Patentblatt 86/01**

(45) Bekanntmachung des Hinweises auf die Patenterteilung : **11.01.89 Patentblatt 89/02**

(84) Benannte Vertragsstaaten :
**AT BE CH DE FR GB IT LI LU NL SE**

(56) Entgegenhaltungen :
FR-A- 2 312 233
FR-A- 2 331 325

(73) Patentinhaber : **Wella Aktiengesellschaft**
**Berliner Allee 65**
**D-6100 Darmstadt (DE)**

(72) Erfinder : **Hoch, Dietrich**
**Ringstrasse 48**
**D-6102 Pfungstadt (DE)**
Erfinder : **Konrad, Eugen**
**Mecklenburger Strasse 101**
**D-6100 Darmstadt (DE)**
Erfinder : **Pasquier, Gilbert**
**Rte. du Centre 10**
**CH-1723 Marly (CH)**
Erfinder : **Mager, Herbert, Dr.**
**Beaumont 5**
**CH-1700 Fribourg (CH)**

**EP 0 166 100 B1**

**Beschreibung**

Oxidationshaarfärbemittel in Cremeform haben gegenwärtig eine besondere Bedeutung erlangt. Solche Haarfärbemittel enthalten im allgemeinen als wichtigste Oxidationsfarbstoffe p-substituierte Benzolderivate, wie zum Beispiel 1,4-Diaminobenzol, 2,5-Diaminotoluol, p-Aminophenol und 2,5-Diaminobenzylalkohol. Diese werden als Entwicklersubstanzen bezeichnet. Die Entwicklersubstanzen müssen in Kombination mit geeigneten Kupplersubstanzen eingesetzt werden. Als Kupplersubstanzen kommen insbesondere bestimmte m-substituierte Benzolderivate oder bestimmte Pyridinderivate zum Einsatz. Beispiele für übliche Kupplersubstanzen sind Resorcin, 4-Chlorresorcin, 4-Hydroxy-1,2-methylendioxybenzol, 4-Amino-1,2-methylendioxybenzol, 2-Methylresorcin, 2,4-Dihydroxyphenolether, 4-Hydroxyindol, 1-Naphthol, m-Aminophenol, m-Phenylendiamin und m-Phenylendiaminderivate.

Übliche Oxidationshaarfärbemittel sind, vorzugsweise durch einen Zusatz an Ammoniak oder Monoethanolamin, alkalisch eingestellt.

Die Entwicklersubstanzen und Kupplersubstanzen liegen häufig in Form ihrer Chloride oder Sulfate vor, wodurch nach dem Zusatz von Ammoniak die entsprechenden Ammoniumsalze gebildet werden. Diese Salze wirken bei üblichen cremeförmigen Oxidationshaarfärbemitteln, gemeinsam mit den Entwicklern, den Kupplern und den gegebenenfalls noch enthaltenen direktfärbenden Farbstoffen, der Bildung der Emulsion entgegen und verringern ihre Stabilität. Besonders bei dunklen farbstoffreichen Haarfärbezusammensetzungen besteht daher stets die Gefahr einer Emulsionstrennung.

Bisher war es erforderlich, die Konsistenz üblicher cremeförmiger Oxidationshaarfärbemittel auf eine hohe Viskosität (oberhalb von etwa 10 000 mPa.s, Stab II/30 °C/20 g) einzustellen. Dadurch wurden Trennerscheinungen vermieden, und es war die Möglichkeit gegeben, die Emulsion durch hohe Anteile an Farbstoffen und Elektrolyten zu belasten.

In den vorstehend geschilderten üblichen Haarfärbemitteln werden zur Erhöhung der Viskosität vor allem Fettalkohole und Fettamide verwendet. Diese Verdicker müssen, um eine gute Emulsionsstabilität zu erhalten, in hohen Konzentrationen von etwa 15 bis 30 Gewichtsprozent eingesetzt werden. Als Emulgatoren finden in üblichen cremeförmigen Haarfärbemitteln oxethylierte Fettalkohole, Fettalkoholsulfate und oxethylierte Fettalkoholsulfate Verwendung.

Ist die Viskosität der Haarfärbemittel hoch, so sind diese Mittel nur schwer im Haar zu verteilen. Sie müssen daher in der Regel mit einem Pinsel aufgetragen werden. Die Auftragung auf das Haar mit Hilfe einer Schüttelflasche und damit eine Anwendung durch den Färbekunden selbst ist nicht möglich. Infolge der hohen Viskosität, welche durch die teilweise Verdunstung von Flüssigkeit während der Färbebehandlung noch zunimmt, ist das vollständige Ausspülen nach der Haarfärbebehandlung erschwert, so daß eine nachfolgende Haarwäsche unerläßlich ist.

Vor dem Beginn der Haarfärbung wird das cremeförmige Oxidationshaarfärbemittel mit einer wäßrigen Hydrogenperoxidlösung, z. B. einer 6 %-igen $H_2O_2$-Lösung, üblicherweise etwa im Verhältnis 1 : 1 vermischt.

Die Hydrogenperoxidlösung kann klar oder getrübt vorliegen, wobei als Trübungsmittel beispielsweise eine Latexdispersion eingesetzt wird.

Ein weiterer Nachteil von üblichen hochviskosen Haarfärbemitteln besteht darin, daß ihre Viskosität während der Lagerung ansteigt, so daß dadurch die Entnahme aus der Tube sehr erschwert wird und die Vermischung mit der Hydrogenperoxidlösung mehr Zeit erfordert.

Aufgabe der vorliegenden Anmeldung war es daher, ein Oxidationshaarfärbemittel zur Verfügung zu stellen, das eine geringere Viskosität besitzt als die vorstehend geschilderten bekannten Mittel, dennoch nicht vom Haar abläuft und ferner nicht die geschilderten Nachteile bekannter cremeförmiger Haarfärbemittel aufweist.

Demgegenüber wurde nunmehr gefunden, daß sich neue niedrigviskose Oxidationshaarfärbemittel mit wesentlich verbesserten Eigenschaften herstellen lassen, bei denen der Zusatz von Farbstoffen und Elektrolyten nicht destabilisierend auf die Emulsion, sondern im Gegenteil stabilisierend bezüglich einer Verhinderung von Phasentrennung sowie einer Beibehaltung der eingestellten Viskosität während der Lagerung wirkt.

Gegenstand der vorliegenden Anmeldung sind daher Mittel zum oxidativen Färben von menschlichen Haaren auf der Basis einer Trägermasse und eines darin gelösten Farbstoffgemisches, dadurch gekennzeichnet, daß die Trägermasse gebildet wird aus

(1) 16 bis 30 Gew.% eines Gemisches von

a) 0,2-5,0 Gew.% mindestens eines physiologisch unbedenklichen wasserlöslischen anorganischen Salzes,

b) 1,4-5,0 Gew.% Natriumlaurylalkoholdiglykolethersulfat,

c) 0,5-6,0 Gew.% Kokosfettsäurediethanolamid,

d) 4,0-14,0 Gew.% eines Gemisches, bestehend aus

60 bis 80 Gewichtsteilen Cetylstearylalkohol

10 bis 30 Gewichtsteilen Glycerin-monodi-stearat und

0 bis 20 Gewichtsteilen Wollwachsalkohol sowie
e) 0,1-2,0 Gew.% quaternisiertes Homopolymerisat von Dimethylaminoethylmethacrylat,

(2) 56 bis 83 Gew.% Wasser,
(3) 0,1 bis 5,0 Gew.% Ammoniak,
(4) 0 bis 5 Gew.% aliphatischem Alkohol,
(5) 0 bis 1 Gew.% Parfümöl und
(6) 0 bis 0,5 Gew.% eines Komplexbildners für Schwermetalle.

Die vorstehenden Gewichtsprozent-Angaben beziehen sich alle jeweils auf die Gesamtmenge des Oxidationshaarfärbemittels.

Die bevorzugt verwendeten Mengen betragen beim anorganischem Salz 1,0 bis 3,0 Gew.%, besonders bevorzugt 2,5 Gew.%. Das Natriumlaurylalkoholdiglykolethersulfat ist vorzugsweise in einer Menge von 2,0 bis 3,5 Gew.% und besonders bevorzugt in einer Menge von 2,8 Gew.% enthalten. Das Kokosfettsäurediethanolamid ist vorzugsweise in einer Menge von 2,0 bis 4,0 Gew.%, insbesondere 3,0 Gew.%, enthalten. Die Komponente d), welche im weiteren als Wachsgemisch bezeichnet wird, ist bevorzugt in einer Menge von 10,0 bis 14,0 Gew.% und besonders bevorzugt in einer Menge von 12 Gew.% enthalten. Der Wassergehalt der Trägermasse liegt bei 56 bis 83 Gew.% und beträgt vorzugsweise etwa 60 bis 70 Gew.%. Das quaternisierte Homopolymerisat von Dimethylaminoethylmethacrylat, welches vorzugsweise zu 75 %, z. B. mit Dimethylsulfat, quaternisiert ist, soll insbesondere in einer Menge von 0,1 bis 0,5 Gew.% und besonders bevorzugt in einer Menge von 0,25 Gew.% enthalten sein.

Die Trägermasse der erfindungsgemäßen Mittel ist durch einen Gehalt an etwa 0,1 bis 5,0 Gew.% Ammoniak auf einen pH-Wert von etwa 8,0 bis 11,5 eingestellt.

Als physiologisch unbedenkliche wasserlösliche anorganische Salze kommen insbesondere Ammonium-, Natrium- oder Kalium-sulfit, -sulfat oder -chlorid in Betracht, wobei die Gesamtmenge dieser in dem Mittel enthaltenen Salze vorzugsweise 1,0 bis 3,0 Gew.% beträgt.

Das Natriumlaurylalkoholdiglykolethersulfat kann z. B. in Form einer handelsüblichen 28 %-igen wäßrigen Lösung vorliegen. Beispiele für geeignete Cetylstearylalkohole sind die Handelsprodukte Lanette Wᵉ und Lanette O der Firma Henkel, Düsseldorf. Als geeignetes Glycerin-mono-distearat kann beispielsweise ein solches mit 30 bis 35 Gew.% Monoestergehalt eingesetzt werden (z. B. Handelsprodukt Teginᵉ der Firma Goldschmidt, Essen, Deutschland). Als Beispiel für ein handelsübliches quaternisiertes Homopolymerisat von Dimethylaminoethylmethacrylat sei das Handelsprodukt PLEX 7525L der Firma Röhm, Darmstadt angeführt.

Gegebenenfalls können die Oxidationshaarfärbemittel als aliphatischen Alkohol z. B. Ethanol oder Isopropanol in einer Menge bis zu 5 Gew.% sowie als Komplexbildner für Schwermetalle beispielsweise Ethylendiamintetraacetat und Nitrilotriessigsäure in einer Menge bis zu 0,5 Gew.% enthalten. Parfümöle können in den Mitteln bis zu einer Menge von etwa 1 Gew.% enthalten sein.

Das in den Oxidationshaarfärbemitteln enthaltene Farbstoffgemisch besteht aus mindestens einer Kupplersubstanz und mindestens einer Entwicklersubstanz sowie gegebenenfalls zusätzlich mit sich selbst kuppelnden Farbvorstufen und direkt auf das Haar aufziehenden Farbstoffen.

Die Entwickler- und Kupplersubstanzen werden in den Haarfärbemitteln entweder als solche oder in Form ihrer physiologisch unbedenklichen Salze mit anorganischen oder organischen Säuren, wie zum Beispiel als Chlorid, Sulfat, Phosphat, Acetat, Propionat, Lactat oder Citrat, eingesetzt.

Die Kupplersubstanzen werden im allgemeinen in etwa äquimolarer Menge, bezogen auf die verwendeten Entwicklersubstanzen, eingesetzt. Wenn sich auch der äquimolare Einsatz als zweckmäßig erweist, so ist es doch nicht nachteilig, wenn die Kupplersubstanzen in einem gewissen Überschuß oder Unterschuß zum Einsatz kommen. Es ist ferner nicht notwendig, daß die Entwicklerkomponente und die Kupplerkomponente einheitliche Produkte darstellen, vielmehr kann sowohl die Entwicklerkomponente ein Gemisch von bekannten Entwicklersubstanzen als auch die Kupplerkomponente ein Gemisch von bekannten Kupplersubstanzen darstellen.

Die Haarfärbemittel enthalten als bekannte Kupplersubstanzen insbesondere 1-Naphthol, 4-Methoxy-1-naphthol, Resorcin, 4-Chlorresorcin, 4,6-Dichlorresorcin, 2-Methylresorcin, m-Aminophenol, 4-Hydroxy-1,2-methylendioxybenzol, 4-Amino-1,2-methylendioxybenzol, 4-(β-Hydroxyethylamino)-1,2-methylendioxybenzol und 5-Amino-2-methylphenol. Weitere geeignete Kupplersubstanzen sind zum Beispiel 2,4-Dihydroxyphenolether wie 2,4-Dihydroxyanisol und 2,4-Dihydroxyphenoxyethanol.

Von den bekannten Entwicklersubstanzen kommen als Bestandteil der erfindungsgemäßen Haarfärbemittel vor allem 1,4-Diaminobenzol, 2,5-Diaminotoluol, 2,5-Diaminoanisol, 2,5-Diaminobenzylalkohol, 3-Methyl-4-aminophenol, 2-(β-Hydroxyethyl)-1,4-diaminobenzol, Tetraaminopyrimidin und 4-Aminophenol in Betracht.

Die Gesamtmenge der in den hier beschriebenen Haarfärbemitteln enthaltenen Entwicklersubstanz-Kupplersubstanz-Kombination soll etwa 0,1 bis 5,0 Gew.%, insbesondere 0,5 bis 5,0 Gew.%, betragen.

Zur Erzielung gewisser Farbnuancen können ferner auch übliche direktziehende Farbstoffe, beispielsweise Triphenylmethanfarbstoffe wie Diamond Fuchsin (C.I. 42 510) und Leather Ruby HF (C.I. 42 520), aromatische Nitrofarbstoffe wie 2-Amino-4,6-dinitro-phenol, 2-Nitro-4-(β-hydroxyethylamino)-anilin, 2-N-β,γ-Dihydroxypropylamino-5-(N-methyl, N-hydroxyethyl) amino-nitrobenzol und 2-Amino-4-nitrophenol,

3

Azofarbstoffe wie Acid Brown 4 (C.I. 14 805) und Acid Blue 135 (C.I. 13 385), Anthrachinonfarbstoffe wie Disperse Violet 4 (C.I. 61 105), Disperse Blue 1 (C.I. 64 500), Disperse Red 15 (C.I. 60 710), Disperse Violet 1 (C.I. 61 100), außerdem 1,4,5,8-Tetraaminoanthrachinon und 1,4- Diaminoanthrachinon, enthalten sein.

Die Haarfärbemittel können weiterhin auch mit sich selbst kuppelnde Farbvorstufen, wie zum Beispiel 2-Amino-5-methylphenol, 2-Amino-6-methylphenol, 2-Amino-5-ethoxyphenol oder auch 2-Propylamino-5-aminopyridin, enthalten.

Die Gesamtmenge des Farbstoffgemisches beträgt in den hier beschriebenen Mitteln etwa 0,1 bis 5,0 Gew.%, vorzugsweise 0,5 bis 5,0 Gew.%.

Das erfindungsgemäße Oxidationshaarfärbemittel stellt ein Gemisch aus der Trägermasse und dem Farbstoffgemisch dar.

Bei der Anwendung vermischt man die Haarfärbemittel unmittelbar vor dem Gebrauch etwa im Gewichtsverhältnis 5 : 1 bis 1 : 4 mit einem Oxidationsmittel und trägt eine für die Haarfärbebehandlung ausreichende Menge, je nach Haarfülle, im allgemeinen etwa 60 bis 200 g, dieses Gemisches auf das Haar auf. Als Oxidationsmittel zur Entwicklung der Haarfärbung kommt hauptsächlich Hydrogenperoxid, beispielsweise als 6-%ige wäßrige Lösung bzw. dessen Additionsverbindungen an Harnstoff, Melamin oder Natriumborat in Betracht. Man läßt das Gemisch bei 15 bis 50 °C etwa 10 bis 45 Minuten lang, vorzugsweise 30 Minuten lang, auf das Haar einwirken, spült sodann das Haar mit Wasser aus und trocknet. Gegebenenfalls wird im Anschluß an diese Spülung mit einer schwachen physiologisch verträglichen organischen Säure, wie zum Beispiel Zitronensäure oder Weinsäure, nachgespült.

Auf Grund der verhältnismäßig geringen Viskosität des Haarfärbemittels, welche etwa 400 bis 4 000, bevorzugt 1 000, mPa.s bei 30 °C (gemessen mit der Viskowaage von Haake mit Stab II und einem Auflagegewicht von 20 Gramm) beträgt, wobei außerdem das Haarfärbemittel nicht homogenisiert zu werden braucht, sowie des hohen Netzmittelgehaltes (Natriumlaurylalkoholdiglykolethersulfat), ist das erfindungsgemäße Haarfärbemittel im Gegensatz zu üblichen cremeförmigen Haarfärbemitteln sehr leicht und rasch mit der Hydrogenperoxidlösung mischbar. Sie wird nach dem Vermischen mit einem Pinsel oder bevorzugt mit der Auftrageflasche auf das zu färbende Haar aufgetragen.

Bei der Färbung des Haarnachwuchses wird zunächst nur der Haaransatz mit dem Gemisch aus Haarfärbemittel und Hydrogenperoxidlösung behandelt. Nach etwa 5 bis 40 Minuten Einwirkzeit bei 15 bis 50 °C wird sodann das Färbegemisch durch Kämmen auf die Haarlängen und Haarspitzen aufgebracht. Das Färbegemisch ist gut im Haar verteilbar. Die dementsprechend geringe mechanische Beanspruchung des Haares beim Kämmen ist besonders bei porösem Haar von Vorteil. Das hier beschriebene Haarfärbemittel wirkt zudem entwirrend und glättend auf das Haar, wodurch eine Verteilung des Mittels im Haar ebenfalls erleichtert wird.

Nach der Einwirkung des Haarfärbemittels kann dieses leicht wieder aus dem Haar gespült werden, so daß ein Waschen der Haare nicht mehr erforderlich ist.

Durch die Anwendung des vorstehend beschriebenen Haarfärbemittels wird das in der Regel poröse Haar gleichzeitig entwirrt und erhält einen angenehmen glatten Griff, so daß es sich auch ohne die zusätzliche Anwendung eines Kurmittels oder einer Haarspülung sehr gut durchkämmen läßt.

Bei den erfindungsgemäßen Haarfärbemitteln ist ein Andicken auch nach längerer Lagerung nicht festzustellen. Die Mittel haben weiterhin die überraschende Eigenschaft, daß sie weitgehend unabhängig von der Art und der Menge des enthaltenen Farbstoff- oder Elektrolytzusatzes die gleiche Endviskosität ergeben. Hierdurch ist es möglich, für alle Farbtöne einer Haarfarbserie die gleiche Trägermasse zu verwenden. Dies erleichtert die Herstellung einer Haarfarbserie erheblich. Bei cremeförmigen Oxidationshaarfärbemitteln nach dem Stande der Technik muß demgegenüber die Trägermasse für die jeweiligen Farbtöne unterschiedlich beschaffen sein. So ist z. B. in dunklen Tönen handelsüblicher Cremehaarfarben der Gehalt an Verdickungsmitteln höher als in hellen Farbtönen.

Die nachstehenden Beispiele sollen den Gegenstand der vorliegenden Erfindung näher erläutern.

### Beispiele

| Beispiel 1 (Trägermasse) | Beispiel 2 | (schwarze Nuancen) |
|---|---|---|
| 0,50 g | 0,50 g | Natriumsulfit, wasserfrei |
| 1,00 g | 2,00 g | Natriumsulfat, wasserfrei |
| 11,00 g | 10,00 g | Natriumlaurylalkoholdiglykolethersulfat (28 %-ige wäßrige Lösung) |
| 2,00 g | 3,00 g | Kokosfettsäurediethanolamid |
| 14,00 g | 12,00 g | eines Gemisches aus 70 Gewichtsteilen Cetylstearylalkohol, 22 Gewichtsteilen Glycerin-mono-distearat und 8 Gewichtsteilen Wollwachsalkohol |
| 0,35 g | 0,25 g | Polydimethylaminoethylmethacrylat, zu 70 % quaternisiert |
| 2,50 g | 2,50 g | Ammoniak |
| 63,75 g | 64,85 g | Wasser |
| 95,10 g | 95,10 g | |

EP 0 166 100 B1

(Fortsetzung)

(Farbstoffgemisch)

| | | |
|---|---|---|
| 0,20 g | 0,20 g | m-Aminophenol |
| 0,50 g | 0,50 g | 2.4-Diaminophenetolsulfat |
| 3,00 g | 3,00 g | 2,5-Diaminotoluolsulfat |
| 1,20 g | 1,20 g | Resorcin |
| 4,90 g | 4,90 g | |
| 100,00 g | 100,00 g | |

40 g des cremeförmigen Haarfärbemittels nach Beispiel 1 oder 2 werden mit 40 g einer üblichen 6 %-igen wäßrigen Hydrogenperoxidlösung vermischt. Die vollständige Durchmischung erfolgt in wenigen Sekunden.

Das Gemisch wird auf den grauen Haarnachwuchs von 6 Wochen zuvor schwarz gefärbten grauen Humanhaaren aufgetragen. Nach einer Einwirkungszeit von 20 Minuten bei 45 °C wird das Haarfärbegemisch durch Kämmen auch in die Haarlängen und Haarspitzen verteilt. Durch die leichte Verteilbarkeit des erfindungsgemäßen Mittels ist dies sehr schnell und mit größtmöglicher Haarschonung möglich. Das Gemisch wird nochmals einige Minuten bei Raumtemperatur einwirken gelassen. Schließlich wird das Haarfärbegemisch mit warmen Wasser aus dem Haar gespült, wobei das Haar nicht nachshampooniert wird.

Das so behandelte Haar ist vom Ansatz bis zu den Haarspitzen gleichmäßig schwarz gefärbt. Das Haar ist sowohl im nassen als auch im trockenen Zustand sehr gut kämmbar und frisierbar. Darüberhinaus weist es einen angenehmen Griff auf. Es sind keine Reste des Färbegemisches im Haar festzustellen.

Die Haarfärbemittel nach Beispiel 1 und 2 verändern ihre Viskosität im Temperaturbereich von 5 bis 40 °C auch nach langer Lagerung nicht. Eine Phasentrennung ist ebenfalls nicht zu beobachten.

| Beispiel 3 | Beispiel 4 | (hellblonde Nuancen) |
|---|---|---|
| (Trägermasse) | | |
| 1,00 g | 0,50 g | Natriumsulfit, wasserfrei |
| 1,00 g | — | Natriumchlorid |
| — | 2,00 g | Natriumsulfat, wasserfrei |
| 10,00 g | 10,00 g | Natriumlaurylalkoholdiglykolethersulfat (28 %-ige wäßrige Lösung) |
| 3,50 g | 3,00 g | Kokosfettsäurediethanolamid |
| 10,00 g | 12,00 g | eines Gemisches aus 70 Gewichtsteilen Cetylstearylalkohol, 22 Gewichtsteilen Glycerin-mono-distearat und 8 Gewichtsteilen Wollwachsalkohol |
| 1,00 g | 1,00 g | Polydimethylaminoethylmethacrylat, zu 70 % quaternisiert, 25 %-ige wäßrige Lösung |
| 10,00 g | 10,00 g | Ammoniak, 25 %-ige wäßrige Lösung |
| 63,00 g | 61,00 g | Wasser |
| 99,50 g | 99,50 g | |

(Farbstoffgemisch)

| | | |
|---|---|---|
| 0,35 g | 0,35 g | 2,5-Diaminotoluolsulfat |
| 0,15 g | 0,15 g | Resorcin |
| 0,50 g | 0,50 g | |
| 100,00 g | 100,00 g | |

Die Anwendung der cremeförmigen Haarfärbemittel nach den Beispielen 3 und 4 erfolgt wie für die Beispiele 1 und 2 beschrieben, jedoch auf dem grauen Haarnachwuchs eines vor 6 Wochen blondgefärbten grauen Humanhaares.

Nach der Färbebehandlung ist das Haar gleichmäßig blond gefärbt und weist alle vorteilhaften Eigenschaften auf, wie sie vorstehend bei den Beispielen 1 und 2 angegeben wurden. Die Mittel gemäß

5

Beispiel 3 und 4 verändern ihre Viskosität während längerer Lagerung nicht und weisen auch keine Phasentrennung auf.

**Patentansprüche**

1. Mittel zum oxidativen Färben von menschlichen Haaren auf der Basis einer Trägermasse und eines darin gelösten Farbstoffgemisches, dadurch gekennnzeichnet, daß die Trägermasse gebildet wird aus

(1) 16 bis 30 Gew.% eines Gemisches von
   a) 0,2-5,0 Gew.% mindestens eines physiologisch unbedenklichen wasserlöslichen anorganischen Salzes,
      b) 1,4-5,0 Gew.% Natriumlaurylalkoholdiglykolethersulfat,
      c) 0,5-6,0 Gew.% Kokosfettsäurediethanolamid,
      d) 4,0-14,0 Gew.% eines Gemisches bestehend aus
      60 bis 80 Gewichtsteilen Cetylstearylalkohol
      10 bis 30 Gewichtsteilen Glycerin-mono-di-stearat und
      0 bis 20 Gewichtsteilen Wollwachsalkohol sowie
      e) 0,1-2,0 Gew.% quaternisiertes Homopolymerisat von Dimethylaminoethylmethacrylat,
(2) 56 bis 83 Gew.% Wasser,
(3) 0,1 bis 5,0 Gew.% Ammoniak,
(4) 0 bis 5 Gew.% aliphatischem Alkohol,
(5) 0 bis 1 Gew.% Parfümöl und
(6) 0 bis 0,5 Gew.% eines Komplexbildners für Schwermetalle,
wobei sich die vorstehenden Gewichtsprozent-Angaben alle jeweils auf die Gesamtmenge des Oxidationshaarfärbemittels beziehen.

2. Mittel zum oxidativen Färben von menschlichen Haaren auf der Basis einer Trägermasse und eines darin gelösten Farbstoffgemisches, dadurch gekennzeichnet, daß die Trägermasse gebildet wird aus

(1) 16 bis 30 Gew.% eines Gemisches von
   a) 1,0-3,0 Gew.% eines physiologisch unbedenklichen wasserlöslichen anorganischen Salzes,
   b) 2,0-3,5 Gew.% Natriumlaurylalkoholdiglykolethersulfat,
   c) 2,0-4,0 Gew.% Kokosfettsäurediethanolamid,
   d) 10,0-14,0 Gew.% eines Gemisches, bestehend aus
   60-80 Gewichtsteilen Cetylstearylalkohol
   10-30 Gewichtsteilen Glycerin-mono-di-stearat und
   0-20 Gewichtsteilen Wollwachsalkohol sowie
   e) 0,1-0,5 Gew.% quaternisiertes Homopolymerisat von Dimethylaminoethylmethacrylat,
(2) 56 bis 83 Gew.% Wasser,
(3) 0,1 bis 5,0 Gew.% Ammoniak,
(4) 0 bis 5 Gew.% aliphatischer Alkohol,
(5) 0 bis 1 Gew.% Parfümöl und
(6) 0 bis 0,5 Gew.% eines Komplexbildners für Schwermetalle,
wobei sich die vorstehenden Gewichtsprozent-Angaben alle jeweils auf die Gesamtmenge des Oxidationshaarfärbemittels beziehen.

3. Mittel nach Anspruch 1 oder 2, bestehend aus
0,1-5,0 Gew.% eines Farbstoffgemisches,
2,5 Gew.% eines physiologisch unbedenklichen wasserlöslichen anorganischen Salzes,
2,8 Gew.% Natriumlaurylalkoholdiglykolethersulfat,
3,0 Gew.% Kokosfettsäurediethanolamid
8,4 Gew.% Cetylstearylalkohol
2,6 Gew.% Glycerin-mono-di-stearat
1,0 Gew.% Wollwachsalkohol
0,25 Gew.% quaternisiertes Homopolymerisat von Dimethylaminoethylmethacrylat,
2,5 Gew.% Ammoniak
73,35-76,45 Gew.% Wasser

4. Mittel nach Anspruch 1 bis 3, dadurch gekennzeichnet, daß das physiologisch unbedenkliche wasserlösliche anorganische Salz ausgewählt ist aus Ammonium-, Natrium- oder Kalium-sulfit, -sulfat oder -chlorid.

5. Mittel nach den Ansprüchen 1 bis 4, dadurch gekennzeichnet, daß das Farbstoffgemisch mindestens eine der Entwicklersubstanzen 1,4-Diaminobenzol, 2,5-Diaminotoluol, 2,5-Diaminobenzylalkohol, 4-Aminophenol, 3-Methyl-4-aminophenol, 2-(β-Hydroxyethyl)-1,4-diaminobenzol, 2,5-Diaminoanisol und Tetraaminopyrimidin enthält.

6. Mittel nach den Ansprüchen 1 bis 5, dadurch gekennzeichnet, daß das Farbstoffgemisch mindestens eine der Kupplersubstanzen 1-Naphthol, 4-Methoxy-1-naphthol, Resorcin, 4-Chlorresorcin,

6

4,6-Dichlorresorcin, 2-Methylresorcin, 2,4-Dihydroxy-anisol, 2,4-Dihydroxyphenoxyethanol, 4-Hydroxy-1,2-methylendioxybenzol, 4-Amino-1,2-methylendioxybenzol, 4-(β-Hydroxyethylamino)-1,2-methylendioxybenzol, m-Aminophenol und 5-Amino-2-methylphenol enthält.

7. Mittel nach den Ansprüchen 1 bis 6, dadurch gekennzeichnet, daß die Gesamtmenge der im Farbstoffgemisch enthaltenen Entwicklersubstanz-Kupplersubstanz-Kombination 0,1 bis 5,0 Gew.% beträgt.

8. Mittel nach den Ansprüchen 1 bis 7, dadurch gekennzeichnet, daß das Farbstoffgemisch als Farbkomponente 2-Amino-5-methylphenol, 2-Amino-6-methylphenol, 2-Amino-5-ethoxyphenol oder 2-Propylamino-5-aminopyridin enthält.

9. Mittel nach den Ansprüchen 1 bis 8 dadurch gekennzeichnet, daß das Farbstoffgemisch mindestens einen der direktziehenden Farbstoffe Diamond Fuchsin (C.I. 42 510), Leather Ruby HF (C.I. 42 520), 2-Amino-4,6-dinitrophenol, 2-Nitro-4-(β-hydroxyethylamino)-anilin, 2-N-β,γ-Dihydroxypropylamino-5-(N-methyl, N-hydroxyethyl) amino-nitrobenzol, 2-Amino-4-nitrophenol, Acid Brown 4 (C.I. 14 805), Acid Blue 135 (C.I. 13 385), Disperse Red 15 (C.I. 60 710), Disperse Violet 1 (C.I. 61 100), 1,4,5,8-Tetraaminoanthrachinon und 1,4-Diamino-anthrachinon enthält.

**Claims**

1. A composition for the oxidative colouring of human hair based on a carrier composition and a mixture of dyestuffs dissolved therein, characterised in that the carrier composition consists of
(1) 16 to 30 % by weight of a mixture of
    (a) 0.2 to 5.0 % by weight of at least one physiologically-acceptable water soluble inorganic salt,
    (b) 1.4 to 5.0 % by weight sodium lauryl alcohol diglycolether sulphate,
    (c) 0.5 to 6.0 % by weight coconut oil fatty acid diethanol amide,
    (d) 4.0 to 14.0 % by weight of a mixture consisting of
    60 to 80 parts by weight cetyl stearyl alcohol
    10 to 30 parts by weight glycerol monodistearate and
    0 to 20 parts by weight wool wax alcohol and also
    (e) 0.1 to 2.0 % by weight quaternised homopolymer of dimethyl amino ethyl methacrylate,
(2) 56 to 83 % by weight water,
(3) 0.1 to 5.0 % by weight ammonia,
(4) 0 to 5 % by weight aliphatic alcohol,
(5) 0 to 1 % by weight perfume oil and
(6) 0 to 0.5 % by weight of a complex former for heavy metals,
the above mentioned indications of percentages by weight all relating to the total quantity of oxidative hair dye.

2. A composition for the oxidative colouring of human hair and based on carrier composition and a mixture of dyestuffs dissolved therein, characterised in that the carrier composition consists of
(1) 16 to 30 % by weight of a mixture of
    (a) 1.0 to 3.0 % by weight of a physiologically-acceptable water soluble inorganic salt,
    (b) 2.0 to 3.5 % by weight sodium lauryl alcohol diglycol ether sulphate,
    (c) 2.0 to 4.0 % by weight coconut oil fatty acid diethanol amide,
    (d) 10.0 to 14.0 % by weight of a mixture consisting of
    60-80 parts by weight cetyl stearyl alcohol
    10-30 parts by weight glycerol monodistearate and
    0-20 parts by weight wool wax alcohol and
    (e) 0.1 to 0.5 % by weight quaternised homopolymer of dimethyl amino ethyl methacrylate,
(2) 56 to 83 % by weight water,
(3) 0.1 to 5.0 % by weight ammonia,
(4) 0 to 5 % by weight aliphatic alcohol,
(5) 0 to 1 % by weight perfume oil and
(6) 0 to 0.5 % by weight of a complex former for heavy metals,
to the above indications of percentages by weight all referring to the total quantity of oxidative hair dye.

3. A composition according to claim 1 or 2, consisting of
0.1 to 5.0 % by weight of a mixture of dyestuffs,
2.5 % by weight of a physiologically acceptable water soluble inorganic salt
2.8 % by weight sodium lauryl alcohol diglycol ether sulphate,
3.0 % by weight coconut fatty acid diethanol amide,
8.4 % by weight cetyl stearyl alcohol,
2.6 % by weight glycerol monodistearate,
1.0 % by weight wool wax alcohol,
0.25 % by weight quaternised homopolymer of dimethyl amino ethyl methacrylate,
2.5 % by weight ammonia,
73.35-76.45 % by weight water.

7

EP 0 166 100 B1

4. A composition according to claims 1 to 3, characterised in that the physiologically acceptable water soluble inorganic salt is chosen from ammonium, sodium or potassium sulphite, sulphate or chloride.

5. A composition according to claims 1 to 4, characterised in that the mixture of dyestuffs contains at least one of the developer substances 1,4-diaminobenzene, 2,5-diaminotoluene, 2,5-diamino benzyl alcohol, 4-aminophenol, 3-methyl-4-amino-phenol, 2-(β-hydroxyethyl)-1,4-diaminobenzene, 2,5-diaminoanisole and tetraamino pyrimidine.

6. A composition according to claims 1 to 5, characterised in that the mixture of dyestuffs contains at least one of the coupler substances 1-naphthol, 4-methoxy-1-naphthol, resorcinol, 4-chlororesorcinol, 4,6-dichlororesorcinol, 2-methylresorcinol, 2,4-dihydroxy-anisole, 2,4-dihydroxyphenoxy ethanol, 4-hydroxy-1,2-methylene dioxybenzene, 4-amino-1,4-methylene dioxybenzene, 4-(β-hydroxyethyl amino)-1,2-methylene dioxy-benzene, m-aminophenol and 5-amino-2-methylphenol.

7. A composition according to claims 1 to 6, characterised in that the total quantity of developer substance/coupler substance combination contained in the mixture of dyestuffs amounts to 0.1 to 5 % by weight.

8. A composition according to claims 1 to 7, characterised in that the mixture of dyestuffs contains as a colouring component 2-amino-5-methylphenol, 2-amino-6-methylphenol, 2-amino-5-methylphenol, 2-amino-6-methylphenol, 2-amino-5-ethoxyphenol or 2-propyl-amino-5-amino pyridine.

9. A composition according to claims 1 to 8, characterised in that the mixture of dyestuffs contains at least one of the direct dyestuffs Diamond Fuchsin (C.I. 42 510), Leather Ruby HF (C.I. 42 520), 2-amino-4,6-dinitrophenol, 2-nitro-4-(β-hydroxyethyl amino)-aniline, 2-N-β,γ-dihydroxy propylamino-5-(N-methyl, N-hydroxyethyl) amino-nitrobenzene, 2-amino-4-nitrophenol, Acid Brown 4 (C.I. 14 805), Acid Blue 135 (C.I. 13 385), Disperse Red 15 (C.I. 60 710), Disperse Violet 1 (C.I. 61 100), 1,4,5,8-tetraaminoanthraquinone and 1,4-diamino-anthraquinone.


**Revendications**

1. Composition pour la coloration d'oxydation des cheveux humains à base d'un véhicule et d'un mélange de colorants qui y est dissous, caractérisée en ce que le véhicule est constitué par
(1) 16 à 30 % en poids d'un mélange de
a) 0,2 à 5,0 % en poids d'au moins un sel anorganique soluble dans l'eau physiologiquement neutre,
b) 1,4 à 5,0 % en poids de sulfate d'éther diglycolique de l'alcool laurylique sodé,
c) 0,5 à 6,0 % en poids de diéthanolamide d'acide d'huile de coprah,
d) 4,0 à 14,0 % en poids d'un mélange comprenant
60 à 80 parties en poids d'alcool cétyl-stéarylique,
10 à 30 parties en poids de mono et di-stéarate, de glycérine
0 à 20 parties en poids d'alcool de cire de suint, et
e) 0,1 à 2,0 % en poids d'homopolymérisat quaternisé de méthacrylate de diméthylaminoéthyle,
(2) 56 à 83 % en poids d'eau,
(3) 0,1 à 5,0 % en poids d'ammoniaque,
(4) 0 à 5 % en poids d'alcool aliphatique,
(5) 0 à 1 % en poids d'huile parfumée, et
(6) 0 à 0,5 % en poids d'un complexant pour métaux lourds,
les % en poids précédents se rapportent à la quantité totale de la composition pour la teinture des cheveux.

2. Composition pour la coloration d'oxydation des cheveux humains à base d'un véhicule et d'un mélange de colorants qui y est dissous, caractérisée en ce que le véhicule est constitué par
(1) 16 à 30 % en poids d'un mélange de
a) 1,0 à 3,0 % en poids d'un sel anorganique soluble dans l'eau physiologiquement neutre,
b) 2,0 à 3,5 % en poids de sulfate d'éther diglycolique de l'alcool laurylique sodé,
c) 2,0 à 4,0 % en poids de diéthanolamide d'acide d'huile de coprah,
d) 10,0 à 14,0 % en poids d'un mélange comprenant
60 à 80 parties en poids d'alcool cétyl-stéarylique,
10 à 30 parties en poids de mono-et-di-stéarate de glycérine et
0 à 20 parties en poids d'alcool de cire de suint, et
e) 0,1 à 2,0 parties en poids d'homopolymérisat quaternisé de méthacrylate de diméthylaminoéthyle,
(2) 56 à 83 % en poids d'eau,
(3) 0,1 à 5,0 % en poids d'ammoniaque,
(4) 0 à 5 % en poids d'alcool aliphatique,
(5) 0 à 1 % en poids d'huile parfumée, et
(6) 0 à 0,5 % en poids d'un complexant pour métaux lourds,
les % en poids précédents se rapportent à la quantité totale de la composition pour la teinture des

8

cheveux.

3. Composition selon la revendication 1 ou 2, comprenant 0,5 à 5,0 % en poids d'un mélange de colorants,

2,5 % en poids d'un sel anorganique soluble dans l'eau physiologiquement neutre

2,8 % en poids de sulfate d'éther diglycolique de l'alcool laurylique sodé,

3,0 % en poids de diéthanolamide d'acide d'huile de coprah,

8,4 % en poids d'alcool cétyl-stéarylique,

2,6 % en poids mono-et-di-stéarate de glycérine,

1,0 % en poids d'alcool de cire de suint,

0,25 % en poids d'homopolymérisat quaternisé de méthacrylate de diméthylaminoéthyle,

2,5 % en poids d'ammoniaque,

73,35 à 76,45 % en poids d'eau.

4. Composition selon l'une des revendications 1 à 3, caractérisée en ce que le sel anorganique soluble dans l'eau physiologiquement neutre est choisi parmi les sulfite, sulfate et chlorure d'ammonium, de sodium ou de potassium.

5. Composition selon les revendications 1 à 4, caractérisée en ce que le mélange de colorants comprend au moins l'un des développeurs suivants : le 1,4-diaminobenzène, le 2,5-diaminotoluène, l'alcool 2,5-diaminobenzylique, le 4-aminophénol, le 3-méthyl-4-aminophénol, le 2-(β-hydroxyéthyl)-1,4-diaminobenzène, le 2,5-diaminoanisole et la tétra-aminopyrimidine.

6. Composition selon les revendications 1 à 5, caractérisée en ce que le mélange de colorants comprend l'un au moins des copulants suivants : le 1-naphtol, le 4-méthoxy-1-naphtol, la résorcine, la 4-chloro-résorcine, la 4,6-dichloro-résorcine, la 2-méthyl-résorcine, le 2,4-dihydroxy-anisole, le 2,4-dihydroxyphénoxyéthanol, le 4-hydroxy-1,2-méthylène-dioxybenzène, le 4-amino-1,2-méthylène-dioxybenzène, le 4-(β-hydroxyéthylamino)-1,2-méthylène-dioxybenzène, le m-aminophénol et le 5-amino-2-méthylphénol.

7. Composition selon les revendications 1 à 6, caractérisée en ce que la proportion totale de la combinaison développeur-copulant contenue dans le mélange de colorants est de 0,5 à 5,0 % en poids.

8. Composition selon les revendications 1 à 7, caractérisée en ce que le mélange de colorants comprend, comme composants colorants, le 2-amino-5-méthylphénol, le 2-amino-6-méthylphénol, le 2-amino-5-éthoxyphénol ou la 2-propylamino-5-aminopyridine.

9. Composition selon les revendications 1 à 8, caractérisée en ce que le mélange de colorants comprend l'un au moins des colorants montant directement sur la fibre suivants : Diamond Fuchsin (I.C. 42 510), Leather Ruby HF (I.C. 42 520), le 2-amino-4,6-dinitrophénol, la 2-nitro-4-(β-hydroxyéthylamine)-aniline, le 2-N-β,γ-dihydroxypropylamino-5-(N-méthyl, N-hydroxyéthyl)-amino-nitrobenzène, le 2-amino-4-nitrophénol, Acid Brown 4 (I.C. 14 805), Acid Blue 135 (I.C. 13 385), Disperse Red 15 (I.C. 60 710), Disperse Violet 1 (I.C. 61 100), la 1,4,5,8-tétra-amino-anthraquinone et la 1,4-diamino-anthraquinone.